(19) Europäisches Patentamt / European Patent Office / Office européen des brevets

(11) **EP 2 980 706 A1**

(12) **DEMANDE DE BREVET EUROPEEN**

(43) Date de publication:
03.02.2016 Bulletin 2016/05

(51) Int Cl.:
*G06F 17/18* (2006.01)  *G06F 3/01* (2006.01)
*A61B 5/04* (2006.01)

(21) Numéro de dépôt: **15178478.2**

(22) Date de dépôt: **27.07.2015**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(30) Priorité: **29.07.2014 FR 1457325**

(71) Demandeur: **Commissariat à l'Énergie Atomique et aux Énergies Alternatives 75015 Paris (FR)**

(72) Inventeurs:
• DURAND, Pierre
38700 CORENC (FR)
• LABYT, Etienne
38950 Saint Martin le Vinoux (FR)

(74) Mandataire: **Brevalex**
**56, Boulevard de l'Embouchure**
**B.P. 27519**
**31075 Toulouse Cedex 2 (FR)**

(54) **PROCÉDÉ DE LOCALISATION D'UNE ACTIVITÉ CÉRÉBRALE ASSOCIÉE À UNE TÂCHE**

(57) L'invention concerne une méthode d'estimation de l'activité électrique d'un tissu à l'aide d'une pluralité de capteurs, en particulier de l'activité cérébrale relative à une tâche motrice effectuée, imaginée ou visualisée par un sujet, à l'aide d'une pluralité de capteurs magnétoencéphalographiques ou électro-encéphalographiques, lorsque que ce sujet est soumis à un stimulus. La méthode d'estimation fait appel à un critère MNE dans lequel les coefficients de la matrice de covariance des signaux physiologiques acquis par les différents capteurs sont pondérés à l'aide des coefficients de corrélation de ces signaux avec un signal représentatif du stimulus.

acquisition des signaux physiologiques $y_i(t)$ du tissu par les capteurs $i = 1,...,N$ — 110

calcul des coefficients de corrélation $\chi_n(t)$ des signaux physiologiques avec le signal de stimulus $\eta(t)$ — 120

calcul de la matrice $\mathbf{C}$ de covariance de bruit — 130

calcul de la matrice $\tilde{\mathbf{C}}(t)$ de covariance de bruit pondérée à partir de la matrice $\mathbf{C}$ et des coefficients de corrélation $\chi_n(t)$ — 140

estimation de l'activité électrique dans le tissu
$\hat{\mathbf{x}}(t) = \mathbf{A}^T \left( \mathbf{A}\mathbf{A}^T + p^{-1}\tilde{\mathbf{C}}(t) \right)^{-1} \mathbf{y}(t)$ — 150

FIG. 1

EP 2 980 706 A1

**Description**

**DOMAINE TECHNIQUE**

La présente invention concerne de manière générale l'estimation de l'activité électrique dans un tissu humain ou animal, et plus particulièrement la localisation d'une activité cérébrale à partir de signaux physiologiques, obtenus par magnétoencéphalographie ou par électroencéphalographie. L'invention s'applique notamment au domaine de la neuroimagerie fonctionnelle et de la commande neuronale directe.

**ÉTAT DE LA TECHNIQUE ANTÉRIEURE**

Les méthodes de neuroimagerie fonctionnelle se divisent classiquement entre celles basées sur l'activité métabolique telles que l'imagerie par résonance magnétique fonctionnelle (IRMf), représentant la réponse hémodynamique, ou la tomographie par émission de positrons (TEP), représentant les modifications du débit sanguin, et les méthodes basées sur l'activité électrophysiologique, telles que l'électroencéphalographie (EEG), mesurant l'activité électrique cérébrale au moyen d'électrodes placées sur le scalp du sujet, l'électrocorticographie (ECoG) mesurant l'activité cérébrale au moyen d'électrodes placées directement sur la surface corticale, ou encore la magnétoencéphalographie (MEG) mesurant les champs magnétiques liés à l'activité électrique dans le cerveau.

Les méthodes d'imagerie fonctionnelle EEG et MEG présentent une meilleure résolution temporelle que les méthodes IRMf et TEP. En outre, elles sont non invasives contrairement à l'électrocorticographie. Enfin, la magnétoencéphalographie est particulièrement intéressante en ce que les signaux magnétiques engendrés par les courants dans le cerveau (principalement les courants ioniques dans les dendrites lors de la transmission synaptique), ne subissent pas ou peu de distorsion lorsqu'ils se propagent au travers de la boîte crânienne.

Par ailleurs, l'électroencéphalographie et la magnétoencéphalographie font actuellement l'objet de recherches importantes pour leurs applications potentielles à la commande neuronale directe. La commande neuronale directe ou BCI (*Brain Computer Interface*) permet d'établir une communication directe entre le cerveau d'un utilisateur et un dispositif externe (ordinateur, système électronique, effecteur) sans médiation par une voie musculaire. La commande neuronale directe exploite l'association d'une ou de plusieurs tâches mentales (actions imaginées par le sujet) à une ou plusieurs commandes du dispositif externe. Ainsi une action de la main imaginée par le sujet pourra être associée à un déplacement d'un curseur sur l'écran d'un ordinateur ou le mouvement d'un effecteur. Cette technique est très prometteuse notamment pour des personnes atteintes de paralysie.

Que ce soit dans le domaine de la neuroimagerie fonctionnelle ou dans celui de la commande directe, différentes méthodes ont été mises au point pour localiser une activité cérébrale associée à une tâche (imaginée ou effectuée) à partir de signaux physiologiques acquis par une pluralité de capteurs. Ainsi, dans le cas de l'EEG, les capteurs sont des électrodes permettant d'acquérir des différences de potentiel électriques à la surface du scalp. Dans le cas de la MEG les capteurs (SQUIDs), peuvent être des magnétomètres capables de mesurer l'intensité du champ magnétiques et/ou des gradiomètres planaires (ou axiaux) capables de mesurer le gradient du champ magnétique dans un plan donné. Par exemple, un équipement MEG peut combiner, en une même localisation, trois capteurs élémentaires voire plus : un magnétomètre de précision mesurant l'intensité et l'orientation du champ magnétique en un point et deux gradiomètres planaires, perpendiculaires entre eux mesurant deux composantes du gradient du champ magnétique en ce point.

En tout état de cause, les méthodes précitées ont pour objet de localiser les sources d'activité cérébrale à partir des signaux acquis par les différents capteurs. Plus précisément, si l'on effectue un maillage du cortex en zones élémentaires et si l'on note **x** un vecteur (de taille *M*) représentatif des densités de courant électrique (ou signaux source) dans les différentes zones élémentaires et **y** un vecteur (de taille *N*) représentatif des signaux acquis par les différents capteurs, on a la relation matricielle :

$$\mathbf{y} = \mathbf{A}\mathbf{x} + \mathbf{b} \qquad (1)$$

où **A** est une matrice de taille $N \times M$, dite matrice de transmission (*lead field matrix*) fonction de la zone élémentaire considérée et **b** est un vecteur d'échantillons de bruit de taille *N*. La matrice **A** est généralement obtenue par simulation à partir d'une modélisation du cerveau par éléments frontières ou finis. Ainsi, le cortex est divisé en *M* zones élémentaires et chaque élément du vecteur **x** étant représentatif de la densité de courant électrique dans un voxel, par exemple pris égal à la norme d'un vecteur de densité de courant électrique dans le voxel en question.

La localisation de l'activité cérébrale revient à rechercher le vecteur **x** (ou, à tout le moins un vecteur **x,** dans la mesure où le système (1) est sous-déterminé), à partir du vecteur **y**, c'est-à-dire à inverser la relation (1). Pour cette raison, la localisation de l'activité cérébrale à partir du vecteur de signaux **y** est quelquefois appelée « problème inverse » dans la littérature. Cette inversion est délicate car il existe en fait une infinité de solutions en raison de la sous-détermination du système (1), le nombre de sources étant bien supérieur au nombre de capteurs. On est alors conduit à faire des hypothèses supplémentaires pour pouvoir réaliser l'inversion.

Différentes solutions du problème inverse ont été proposées dans la littérature, notamment la méthode MNE (*Minimum Norm Estimate*), la méthode dSPM (*dynamic Statistical Parameter Mapping*) utilisée dans la localisation de sources profondes, la méthode LORETA (*Low Resolution Electromagnetic Tomography*), les méthodes par formation de faisceau (*Beamforming*) décrites notamment dans l'article de A. Fuchs intitulé « Beamforming and its application to brain connectivity ». publié dans l'ouvrage « Handbook of Brain Connectivity », V.K. Jirsa, R.A. McIntosh, Springer Verlag, Berlin, pp. 357-378 (2007).

On trouvera par ailleurs une revue des différentes méthodes de localisation précitées dans l'article de O. Hauk et al. intitulé « Comparison of noise-normalized minimum norm estimates of MEG analysis using multiple resolution metrics » parue dans Neuroimage, Vol. 54, 2011, pp. 1966-1974.

Si l'on fait l'hypothèse que le bruit est gaussien et plus précisément que les échantillons de bruit sont des variables aléatoires gaussiennes centrées indépendantes et identiquement distribuées, la solution de (1) peut être donnée par la matrice **W** qui minimise l'erreur quadratique :

$$e = \left\| \mathbf{Wy} - \mathbf{x} \right\|^2 \tag{2}$$

L'équation (2) peut encore s'écrire sous la forme :

$$e = \left\| \mathbf{Mx} \right\|^2 + \left\| \mathbf{Wb} \right\|^2 = Tr\left( \mathbf{MRM}^T \right) + Tr\left( \mathbf{WCW}^T \right) \tag{3}$$

où **M** = **WA**-$\mathbf{I}_M$, $\mathbf{I}_M$ est la matrice identité de taille $M \times M$, $Tr$ est la fonction trace, **R** est la matrice de covariance des signaux source, et C est la matrice de covariance du bruit. La minimisation de l'erreur quadratique $e$ conduit à la solution :

$$\mathbf{W} = \mathbf{RA}^T \left( \mathbf{ARA}^T + \mathbf{C} \right)^{-1} \tag{4}$$

et donc à une estimation de la localisation de l'activité cérébrale donnée par :

$$\hat{\mathbf{x}} = \mathbf{RA}^T \left( \mathbf{ARA}^T + \mathbf{C} \right)^{-1} \mathbf{y} \tag{5}$$

La matrice de covariance de bruit peut s'écrire **C** = $\sigma^2 \mathbf{I}_N$ où $\sigma^2$ est la variance du bruit et $\mathbf{I}_N$ est la matrice unité de taille $N \times N$. De même, si les différentes sources sont considérées comme indépendantes et identiquement distribuées (même puissance pour tous les dipôles), on a **R** = $p.\mathbf{I}_M$ où $p$ est la puissance du signal source dans toutes les zones élémentaires, la relation (5) peut se simplifier :

$$\hat{\mathbf{x}} = \mathbf{A}^T \left( \mathbf{AA}^T + \lambda \mathbf{I}_N \right)^{-1} \mathbf{y} \tag{6}$$

où $\lambda = \sigma^2/p$ est un paramètre de régularisation traduisant l'importance du bruit sur le signal source. L'expression (6) est la solution de la méthode MNE mentionnée plus haut.

Quelle que soit la méthode de localisation envisagée, la précision de localisation obtenue décroit rapidement avec le rapport signal sur bruit ($\lambda^{-1}$). Pour pallier cette difficulté, il a été notamment proposé dans l'article de Liu et al. intitulé « Spatiotemporal imaging of human brain activity using functional MRI constrained magnetoencephalography data : Monte Carlo simulations » publié dans Proceedings of the National Academy of Sciences of the United States of America, vol. 95, n° 15, pp. 8945-8950, Juillet 1998, d'exploiter des données de localisation d'IRMf pour améliorer la précision de localisation de l'activité cérébrale MEG ou EEG. Toutefois, cette amélioration de la précision par hybridation de données suppose, d'une part, de disposer d'IRMf pour la même tâche, ce qui n'est pas envisageable pour la commande neuronale directe, et, d'autre part, que l'activité électrique/magnétique dans le cerveau est corrélée avec la réponse hémodynamique. De ce fait, elle introduit inévitablement un biais en orientant la localisation de l'activité cérébrale MEG ou EEG vers des sources qui ont été détectées par IRMf.

Le problème à la base de la présente invention est par conséquent de proposer une méthode de localisation de l'activité cérébrale associée à une tâche, à partir de signaux physiologiques, en particulier de signaux magnétoencéphalographiques ou électroencéphalographiques, qui présente une meilleure précision de localisation que celle obtenue dans l'état de la technique sans recourir à une méthode d'imagerie fonctionnelle tierce. De manière plus générale, le but de la présente invention est de permettre une meilleure estimation de l'activité électrique dans le tissu d'un sujet humain ou animal à partir de signaux physiologiques acquis lorsque le sujet effectue ou évoque une tâche, ce sans information *a priori* sur la localisation de cette activité électrique.

## EXPOSÉ DE L'INVENTION

La présente invention est définie par une méthode d'estimation de l'activité électrique au sein d'un tissu d'un sujet, ladite activité électrique étant associée à une tâche, effectuée, imaginée ou visualisée par le sujet lorsque celui-ci reçoit un stimulus, dans laquelle on réalise l'acquisition d'une pluralité de signaux physiologiques grâce à une pluralité de capteurs disposés autour du tissu, et dans laquelle :

- on calcule des coefficients de corrélation entre les différents signaux physiologiques et un signal représentatif dudit stimulus ;
- on calcule la matrice de covariance des signaux physiologiques sur une fenêtre temporelle ;
- on pondère les coefficients de la matrice de covariance à l'aide des coefficients de corrélation, de sorte à pénaliser, en termes de rapport signal sur bruit, les signaux physiologiques qui sont faiblement corrélés avec le stimulus, la pénalisation diminuant les coefficients relatifs aux signaux physiologiques faiblement corrélés avec le stimulus, lorsque le stimulus est présent dans la fenêtre temporelle et/ou augmentant ces coefficients, lorsque celui-ci est absent de la fenêtre temporelle ;
- on estime l'activité électrique, en au moins un point du tissu, à partir des signaux physiologiques et de la matrice de covariance ainsi pondérée.

L'estimation peut notamment être basée sur un critère MNE.

Dans ce cas, la matrice de covariance est une matrice de covariance de bruit calculée sur une fenêtre temporelle où le stimulus est absent et l'activité électrique dans une pluralité de zones élémentaires du tissu est estimée au moyen de :

$$\hat{\mathbf{x}}(t) = \mathbf{A}^T \left( \mathbf{A}\mathbf{A}^T + p^{-1}\tilde{\mathbf{C}}(t) \right)^{-1} \mathbf{y}(t)$$

où $\hat{\mathbf{x}}(t)$ est un vecteur représentant l'activité électrique dans les différentes zones élémentaires, $\mathbf{y}(t)$ est un vecteur représentant les signaux physiologiques acquis par les capteurs, $\mathbf{A}$ est une matrice donnant la réponse des capteurs pour des sources de puissance unitaire situées dans les différentes zones élémentaires, $p$ est la puissance réelle de ces sources et C(t) est la matrice de covariance de bruit pondérée.

Avantageusement, les coefficients de matrice de covariance de bruit pondérée sont obtenus à partir de la matrice de covariance de bruit au moyen de la relation suivante :

$$\tilde{C}_{ij}(t) = C_{ij} \text{ pour } i = 1,...,N, j = 1,...,N, i \neq j$$

$$\tilde{C}_{ii}(t) = \frac{C_{ii}}{1 + \gamma^2 \chi_i^2(t)} \text{ pour } i = 1,...,N$$

où les coefficients $\tilde{C}_{ij}(t)$, $i=1,...,N$, $j=1,...,N$, sont les coefficients de la matrice de covariance de bruit pondérée, les coefficients $C_{ij}$, $i=1,...,N$, $j=1,...,N$ sont les coefficients de la matrice de covariance de bruit, $N$ est le nombre de capteurs, $\gamma$ est une constante réelle prédéterminé et $\chi_i(t)$, $i=1,...,N$, sont les coefficients de corrélation des signaux physiologiques acquis par les différents capteurs avec le signal représentatif du stimulus.

Les coefficients de corrélation $\chi_i(t)$, $i=1,...,N$ peuvent faire l'objet d'une normalisation préalablement à la pondération des coefficients de la matrice de covariance de bruit.

La méthode d'estimation peut alternativement utiliser une formation de faisceau pour une pluralité de zones élémentaires du tissu et pour une pluralité de directions.

Dans ce cas, la matrice de covariance est calculée sur une fenêtre temporelle où le stimulus est présent et que l'activité électrique dans une chaque zone élémentaire du tissu est estimée au moyen de :

$$\hat{x}_{m,k}(t) = \tilde{\mathbf{D}}(t)^{-1}\mathbf{L}_{m,k}\left(\mathbf{L}_{m,k}\tilde{\mathbf{D}}(t)^{-1}\mathbf{L}_{m,k}\right)^{-1}\mathbf{y}(t)$$

où $\hat{x}_{m,k}(t)$ représente l'activité électrique dans la zone élémentaire située en un point $\mathbf{r}_m$ et dans la direction $\mathbf{u}_k$, $\mathbf{y}(t)$ est un vecteur représentant les signaux physiologiques acquis par les capteurs, $\mathbf{L}_{m,k}$ est un vecteur de taille $N$ donnant la réponse des capteurs lorsqu'une source de puissance unitaire se trouve au point $\mathbf{r}_m$ et est orientée dans la direction $\mathbf{u}_k$, et où $\mathbf{D}(t)$ est la matrice de covariance pondérée.

Avantageusement, les coefficients de matrice de covariance pondérée sont obtenus à partir de la matrice de covariance au moyen de la relation suivante :

$$\tilde{D}_{ij}(t) = D_{ij}\left|\chi_i(t)\right|\left|\chi_j(t)\right| \quad i = 1,...,N, \; j = 1,...,N$$

où les coefficients $\tilde{D}_{ij}(t)$, $i=1,...,N$, $j=1,...,N$, sont les coefficients de la matrice de covariance pondérée, les coefficients $D_{ij}$, $i=1,..., N$, $j =1,..., N$ sont les coefficients de la matrice de covariance, $N$ est le nombre de capteurs et $\chi_i(t)$, $i =1,...,N$, sont les coefficients de corrélation des signaux physiologiques acquis par les différents capteurs avec le signal représentatif du stimulus.

Les coefficients de corrélation peuvent être obtenus en effectuant une transformée temps-fréquence ou temps-échelle de chaque signal physiologique pour obtenir une pluralité de composantes fréquentielles ($Y_f(t)$) de ce signal en fonction du temps, en calculant les coefficients de Pearson ($R_f(t)$) entre lesdites composantes fréquentielles et le signal représentatif du stimulus, le coefficient de corrélation ($\chi(t)$) relatif à un signal physiologique étant déterminé à partir desdits coefficients de Pearson obtenus pour ce signal.

Le coefficient de corrélation ($\chi(t)$) relatif à un signal physiologique peut alors être obtenu comme la valeur extrémale des coefficients de Pearson pour les différentes composantes fréquentielles de ce signal.

## BRÈVE DESCRIPTION DES DESSINS

D'autres caractéristiques et avantages de l'invention apparaîtront à la lecture d'un mode de réalisation préférentiel de l'invention fait en référence aux figures jointes parmi lesquelles :

La Fig. 1 représente de manière schématique l'ordinogramme d'un procédé d'estimation de l'activité électrique dans un tissu selon un premier mode de réalisation de l'invention ;
La Fig. 2 représente de manière schématique l'ordinogramme d'un procédé d'estimation de l'activité électrique dans un tissu selon un second mode de réalisation de l'invention ;
La Fig. 3 représente de manière schématique un exemple de calcul d'un coefficient de corrélation entre un signal physiologique et un signal de stimulus ;
La Fig. 4A représente l'activité électrique dans un cerveau humain, associée à une tâche, estimée à partir d'un procédé connu de l'état de la technique ;
La Fig. 4B représente l'activité électrique dans un cerveau humain, associée à cette même tâche, estimée à partir d'un procédé d'estimation selon un mode de réalisation de l'invention.

## EXPOSÉ DÉTAILLÉ DE MODES DE RÉALISATION PARTICULIERS

On considérera par la suite un système d'acquisition de signaux physiologiques à partir d'une pluralité de capteurs disposés autour d'un tissu d'intérêt d'un sujet, humain ou animal, ce tissu étant le siège d'une activité électrique lorsque ce sujet effectue, voit ou imagine visuellement une tâche. Dans un souci d'illustration et sans préjudice de généralisation nous envisagerons plus particulièrement le cas d'un système d'acquisition magnétoencéphalographique, étant bien entendu que d'autres systèmes d'acquisition pourront être alternativement utilisés, notamment un système d'acquisition électroencéphalographique.

Le système magnétoencéphalographique comprend, de manière connue, un « casque MEG » placé à quelques centimètres de la boite crânienne du sujet. Ce casque comprend une pluralité de capteurs situés en différents points. , chaque capteur pouvant être constitué d'un ou de plusieurs capteurs élémentaires. Les capteurs élémentaires peuvent être des magnétomètres de précision et des gradiomètres planaires (ou axiaux). Alternativement, ils peuvent être des gradiomètres radiaux tels que ceux décrits dans l'article de J. Vrba et al. intitulé « Signal processing in magnetoencephalography », Methods 25, 249-271 (2001). Les mouvements de la tête du sujet sont en outre enregistrés et compensés grâce à des bobines placées en des points fixes par rapport à la tête du sujet et générant un champ magnétique dans une bande de fréquence éloignée de celle du signal MEG.

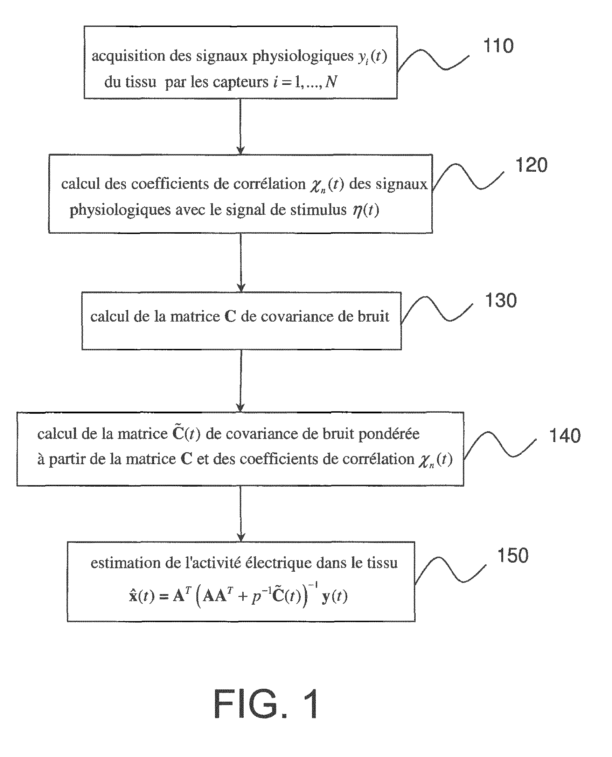

La Fig. 1 représente de manière schématique une méthode d'estimation de l'activité électrique au sein d'un tissu d'un sujet, selon un premier mode de réalisation de l'invention.

A l'étape 110, le sujet effectue, imagine ou visualise une tâche. On a en effet pu montrer que l'imagination ou la visualisation d'une tâche activait la même zone cérébrale que lorsque le sujet effectuait réellement cette tâche.

La tâche en question peut être représentée par une variable binaire $\eta(t)$ indicative d'un stimulus sensoriel, par exemple visuel ou auditif. Par exemple, lorsque la variable $\eta(t)$ prend la valeur 1, le stimulus est appliqué et lorsqu'elle prend la valeur 0, il ne l'est pas. Lorsque le stimulus est appliqué, le sujet effectue, imagine ou visualise la tâche en question.

Le stimulus peut être répété de manière à acquérir, une pluralité de séquences $\mathbf{y}(t)$ où $\mathbf{y}$ est, comme défini précédemment, le vecteur (de dimension $N$) des signaux physiologiques acquis par les différents capteurs au temps $t$).

A l'étape 120, on calcule pour chaque composante $y_n(t)$, $n = 1,...,N$, du vecteur $\mathbf{y}(t)$, un coefficient $\chi_n$ représentatif de la corrélation entre le signal $y_n(t)$ et le stimulus $\eta(t)$, sur une plage temporelle donnée, le coefficient $\chi_n$ étant d'autant plus élevé en valeur absolue que la corrélation entre cette composante et le stimulus est importante dans cette plage temporelle. Le coefficient de corrélation $\chi_n$ peut être obtenu selon différentes variantes, comme décrit plus loin. De manière générale, le coefficient $\chi_n$ dépend de la plage temporelle considérée pour le calcul de la corrélation et par conséquent du temps. Pour cette raison, il sera noté par la suite $\chi_n(t)$.

Au terme de la phase d'apprentissage, on dispose ainsi d'une pluralité de coefficients $\chi_n(t)$, $n = 1,...,N$, indiquant, en fonction du temps, dans quelle mesure les différents signaux physiologiques sont corrélés « avec le stimulus », autrement dit, sont pertinents vis-à-vis de la tâche en question.

A l'étape 130, on calcule la matrice de covariance du bruit, **C**. Cette matrice de covariance de bruit est avantageusement obtenue comme la matrice de covariance des signaux physiologiques $y_n(t)$ en absence d'application du stimulus et de réalisation de tâche par le sujet, en l'occurrence lorsque $\eta(t) = 0$. Autrement dit, la composante $C_{ij}$ de la matrice de covariance est obtenue par :

$$C_{ij} = E\left[ (\mathbf{y}_i - E(\mathbf{y}_i))(\mathbf{y}_j - E(\mathbf{y}_j))^T \right] \text{ lorsque } \qquad \eta(t) = 0 \qquad (7)$$

où E(.) signifie l'espérance mathématique. L'espérance mathématique E(Z) peut être estimée à partir de la moyenne de Z sur l'intervalle de temps pendant lequel le stimulus est absent.

A l'étape 140, on pondère les coefficients (ici les termes diagonaux) de la matrice de covariance de bruit au moyen des coefficients de corrélation précités, de manière à pénaliser, en termes de rapport signal sur bruit, les signaux physiologiques présentant une faible corrélation avec ce stimulus. Cette pondération favorise corrélativement, en termes de rapport signal sur bruit, les signaux physiologiques présentant une corrélation élevée avec le stimulus. La pénalisation se traduit par une augmentation des coefficients de la matrice de covariance relatifs aux signaux physiologiques faiblement corrélés avec le stimulus, dans la mesure où la matrice de covariance est celle de la covariance de bruit.

Cette pondération est dynamique dans la mesure où les coefficients de pondération varient en fonction du temps. Le résultat de cette pondération est une matrice de covariance pondérée notée **C**($t$). Par exemple, les coefficients de la matrice **C**($t$) pourront être obtenus à partir des coefficients de la matrice de covariance de bruit **C**, de la manière suivante :

$$\tilde{C}_{ij}(t) = C_{ij} \text{ pour } i = 1,...,N, j = 1,...,N, i \neq j$$

$$\tilde{C}_{ii}(t) = \frac{C_{ii}}{1 + \gamma^2 \chi_i^2(t)} \text{ pour } i = 1,...,N \qquad (8)$$

où $\gamma$ est un coefficient prédéterminé. Avantageusement, les coefficients de corrélation $\chi_i(t)$, $i = 1,...,N$, sont normalisés :

$$\chi_i(t) = \frac{\chi_i(t) - \min(\chi_i)}{\max(\chi_i) - \min(\chi_i)} \tag{9}$$

de sorte qu'ils prennent leurs valeurs dans l'intervalle [0,1]. D'autres fonctions de pondération pourront être envisagées par l'homme du métier sans sortir du cadre de la présente invention.

A l'étape, 150, on effectue, à chaque instant, une estimation de l'activité électrique dans le tissu à partir du vecteur des signaux physiologiques **y,** de la matrice **A** de transmission du champ ainsi que de la matrice de covariance de bruit pondérée, C :

$$\hat{\mathbf{x}}(t) = \mathbf{R}\mathbf{A}^T \left( \mathbf{A}\mathbf{R}\mathbf{A}^T + \tilde{\mathbf{C}}(t) \right)^{-1} \mathbf{y}(t) \tag{10}$$

où **R** est la matrice de covariance des signaux source relatifs aux différentes zones élémentaires.

On rappelle que la matrice de transmission **A** est obtenue par simulation, préalablement à l'acquisition des signaux physiologiques, en effectuant un maillage du tissu en *M* zones élémentaires et en calculant par une méthode d'éléments frontières ou finis le champ aux points de mesure des différents capteurs. Plus précisément, à partir du champ généré par une source dans une zone élémentaire, on calcule le champ en ces points de mesure. Le processus est répété pour les *M* zones élémentaires de sorte que l'on obtient successivement les *M* lignes de la matrice **A**.

La matrice de covariance **R** peut aussi être obtenue par simulation à partir des signaux source générés dans les différentes zones élémentaires. Avantageusement, on assimile ces signaux source à des variables aléatoires indépendantes et identiquement distribuées (hypothèse généralement vérifiée). Dans ce cas, l'estimation de l'activité électrique dans le tissu est plus simplement donnée par :

$$\hat{\mathbf{x}}(t) = \mathbf{A}^T \left( \mathbf{A}\mathbf{A}^T + p^{-1}\tilde{\mathbf{C}}(t) \right)^{-1} \mathbf{y}(t) \tag{11}$$

où *p* est la puissance du signal source dans une zone élémentaire.

L'expression (11) suppose d'inverser une matrice à chaque plage temporelle considérée. En pratique, on pourra se contenter de n'effectuer cette inversion que tous les $N_f$ fenêtres temporelles ($N_f$ étant un entier supérieur à 1) en remplaçant dans l'expression (8) les coefficients de corrélation par leurs moyennes respectives sur $N_f$ fenêtres temporelles.

Dans tous les cas, l'activité électrique ainsi estimée peut être représentée sous forme d'image pour localiser l'activité ou bien traitée, dans le cas d'une activité cérébrale, pour générer une commande neuronale directe. Dans ce dernier cas, le traitement en question pourra comprendre l'intégration du module du vecteur $\hat{\mathbf{x}}$ sur une zone prédéterminée du cerveau et la comparaison du résultat d'intégration à un seuil, ou encore une corrélation spatiale du vecteur $\hat{\mathbf{x}}$ avec un pattern prédéterminé.

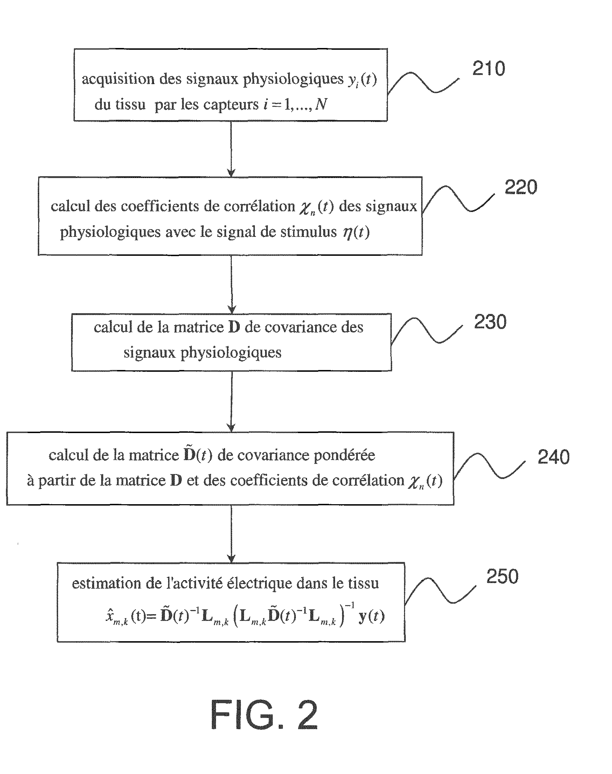

La Fig. 2 représente de manière schématique une méthode d'estimation de l'activité électrique au sein d'un tissu d'un sujet, selon un second mode de réalisation de l'invention.

Les étapes 210 et 220 sont respectivement identiques aux étapes 110 et 120 précédemment décrites. Autrement dit, l'acquisition des signaux physiologiques au moyen des différents capteurs est réalisée en 210 et le calcul des coefficients de corrélation est effectué en 220.

A l'étape 230, on effectue le calcul de la matrice de covariance des signaux physiologiques, notée ci-après D. Au contraire du premier mode de réalisation, le calcul de la matrice de covariance est effectué sur une plage temporelle dans laquelle le stimulus est présent :

$$D_{ij} = E\left[ (\mathbf{y}_i - E(\mathbf{y}_i))(\mathbf{y}_j - E(\mathbf{y}_j))^T \right] \text{ lorsque } \eta(t) = 1 \tag{12}$$

A l'étape 240, on pondère les éléments de la matrice de covariance des signaux physiologiques par les coefficients de corrélation $\chi_n(t)$, *n* =1,...,*N*. Plus précisément on calcule les composantes d'une matrice $\bar{\mathbf{D}}(t)$ :

$$\tilde{D}_{ij}(t) = D_{ij} \left| \chi_i(t) \right| \left| \chi_j(t) \right| \quad i = 1, ..., N, \ j = 1, ..., N \tag{13}$$

Ainsi, dans cette matrice, les coefficients sont pondérés en fonction de la pertinence des signaux physiologiques vis-à-vis de la tâche, les signaux peu pertinents (faibles coefficients de corrélation) étant ici pénalisés en réduisant les coefficients correspondants dans la matrice de corrélation.

A l'étape 250, on effectue formation d'une pluralité de faisceaux, chaque faisceau correspondant à une zone élémentaire du tissu et à une direction d'observation donnée.

On peut montrer (cf. article de A. Fuchs précité) que le signal issu d'une zone élémentaire en un point $\mathbf{r}_m$ (dipôle équivalent dans le cas de la MEG) et observé dans une direction $\mathbf{u}_k$ est obtenu par :

$$x_{m,k}(t) = \mathbf{w}_{m,k}^T \mathbf{y}(t) \tag{14}$$

où $\mathbf{w}_{m,k}$ est un vecteur colonne donnant les poids à affecter à chaque signal physiologique pour la formation de faisceau en réception dans la direction $\mathbf{u}_k$, le vecteur $\mathbf{w}_{m,k}$ étant obtenu par l'expression:

$$\mathbf{w}_{m,k}^T = \mathbf{D}^{-1} \mathbf{L}_{m,k} \left( \mathbf{L}_{m,k} \mathbf{D}^{-1} \mathbf{L}_{m,k} \right)^{-1} \tag{15}$$

dans laquelle le vecteur $\mathbf{L}_{m,k}$ de taille $N$ est le vecteur du champ mesuré par les $N$ capteurs lorsqu'une source se trouve au point $\mathbf{r}_m$ et est orientée dans la direction $\mathbf{u}_k$. Ce vecteur est obtenu par simulation à partir d'un modèle de propagation dans le tissu, de manière connue en soi.

Dans le présent mode de réalisation, on pondère les contributions des signaux physiologiques par les coefficients de corrélation, plus précisément l'activité électrique du tissu au point $\mathbf{r}_m$ dans la direction $\mathbf{u}_k$ est estimée par :

$$\hat{x}_{m,k}(t) = \tilde{\mathbf{w}}_{m,k}^T(t) \mathbf{y}(t) \tag{16}$$

où

$$\tilde{\mathbf{w}}_{m,k}^T(t) = \tilde{\mathbf{D}}(t)^{-1} \mathbf{L}_{m,k} \left( \mathbf{L}_{m,k} \tilde{\mathbf{D}}(t)^{-1} \mathbf{L}_{m,k} \right)^{-1} \tag{17}$$

L'expression (17) nécessite d'inverser la matrice de covariance pondérée $\tilde{\mathbf{D}}(t)$. Comme dans le premier mode de réalisation, cette inversion ne peut être réalisée que toutes les $N_f$ fenêtres temporelles, les coefficients de corrélation dans l'expression (13) étant alors remplacés par leurs moyennes respectives sur ces $N_f$ fenêtres.

L'inversion de la matrice $\mathbf{D}(t)$ peut être réalisée après diagonalisation. Les espaces propres pour lesquelles les valeurs propres sont significatives (supérieures à une valeur de seuil) sont ceux pertinents pour la tâche effectuée ou imaginée par le sujet. On pourra améliorer le conditionnent de la matrice $\tilde{\mathbf{D}}(t)$ à l'aide d'un paramètre de régularisation lorsque les valeurs propres sont inférieures à un seuil déterminé.

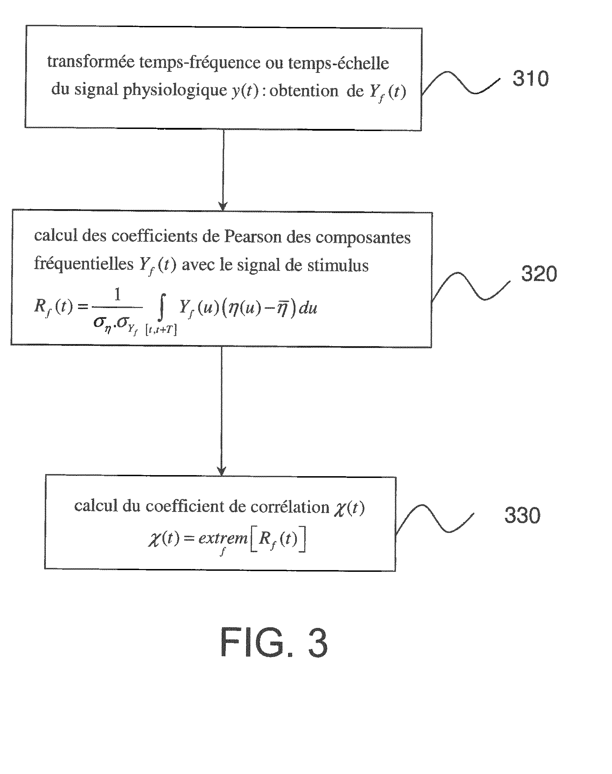

La Fig. 3 représente de manière schématique un exemple de calcul du coefficient de corrélation d'un signal physiologique avec un signal de stimulus, c'est-à-dire, plus précisément, d'un signal $y_n(t)$ fourni par un capteur et le stimulus $\eta(t)$, tel que défini plus haut. Le signal physiologique $y_n(t)$ sera noté dans la suite simplement $y(t)$, le calcul étant identique quel que soit le capteur.

Dans une première étape, 310, on calcule une transformée temps-fréquence ou une transformée temps-échelle du signal physiologique $y(t)$. La transformée temps-fréquence peut être par exemple une transformée de Fourier court-terme par pondération à l'aide d'une fenêtre temporelle glissante, la transformée temps-échelle peut être une transformée par ondelettes ou CWT (*Continuous Wavelet Transform*), de manière connue en soi. On pourra utiliser à cette fin une ondelette de Morlet-Gabor ou une ondelette dite en chapeau mexicain.

Dans tous les cas, on obtient une représentation fréquentielle en fonction du temps, $Y_f(t)$, du signal physiologique $y(t)$, le terme $Y_f(t)$ donnant la composante fréquentielle (ou plus précisément dans une bande fréquentielle) « instantanée » du signal $y(t)$.

Le cas échéant ces composantes fréquentielles peuvent être lissées dans le temps par un filtrage passe-bas, par exemple au moyen d'une moyenne glissante avec coefficient d'oubli.

Dans une seconde étape, 320, on calcule le coefficient de Pearson $R_f$ de chaque composante fréquentielle $Y_f$ de la manière suivante :

$$R_f(t) = \frac{1}{\sigma_\eta . \sigma_{Y_f}} \int_{[t,t+T]} Y_f(u)\big(\eta(u) - \overline{\eta}\big) du \qquad (18)$$

l'intégration sur la fenêtre glissante $[t,t+T]$ pouvant bien entendu être réalisée au moyen d'une sommation discrète et le calcul étant effectué pour un ensemble discret de fréquence. $\sigma_\eta$ et $\sigma_{Y_f}$ représentent respectivement la variance du stimulus $\eta$ et de la composante fréquentielle $Y_f$ et $\overline{\eta}$ est la valeur moyenne de $\eta$ sur la fenêtre glissante en question.

Dans une troisième étape, 330, on calcule le coefficient de corrélation $\chi(t)$ du signal physiologique $\mathbf{y}(t)$ avec le stimulus $\eta(t)$ à partir des coefficients de Pearson $R_f(t)$. Par exemple, on pourra prendre pour $\chi(t)$ la valeur extrémale de $R_f(t)$ dans la plage de fréquence d'intérêt :

$$\chi(t) = \underset{f}{extrem}\Big[ R_f(t) \Big] \qquad (19)$$

On rappelle que la valeur extrémale d'une fonction est celle de la valeur maximale et de la valeur minimale qui est la plus grande en valeur absolue. De manière équivalente, puisqu'il n'intervient précédemment qu'en valeur absolue, le coefficient de corrélation pourra être choisi comme le maximum de $|R_f(t)|$ dans la plage de fréquence d'intérêt. D'autres variantes de calcul du coefficient de corrélation pourront être envisagées par l'homme du métier, par exemple l'intégration de $|R_f(t)|$ ou de $(R_f(t))^2$ sur la plage de fréquence d'intérêt.

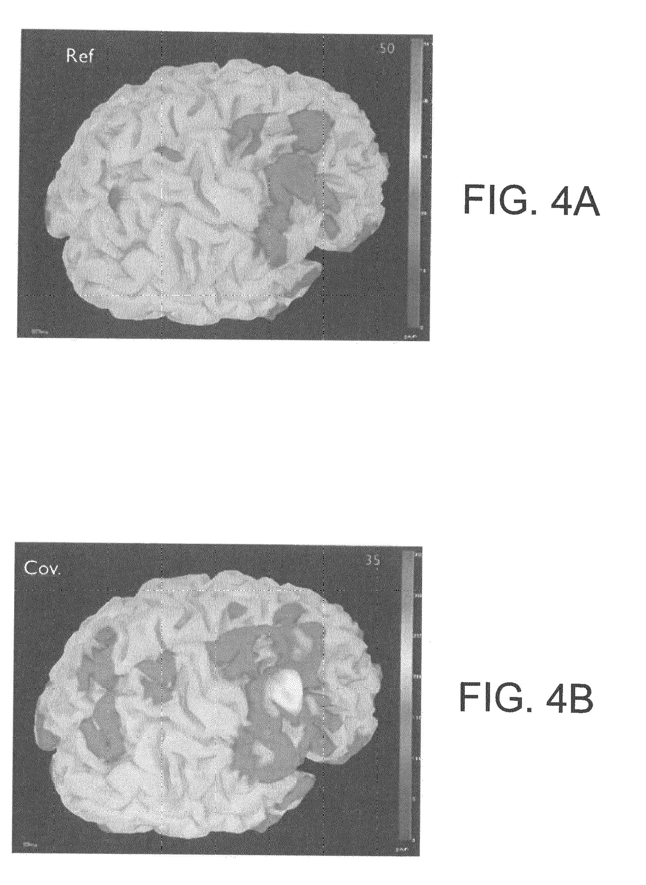

La Fig. 4A représente une image de l'activité électrique dans un cerveau d'un sujet humain, correspondant à une tâche effectuée par ce sujet, telle qu'obtenue par la méthode d'estimation MNE, plus précisément comme estimée au moyen de l'expression (6).

La Fig. 4B représente l'activité électrique dans le cerveau en question, toujours pour la même tâche effectuée, mais obtenue au moyen de la méthode d'estimation selon le premier mode de réalisation de l'invention, plus précisément comme estimée au moyen de l'expression (11).

On remarque que l'activité cérébrale représentée en Fig. 4B est mieux focalisée que celle représentée en Fig. 4A. Ainsi, on a pu sans apport de données de localisation issues d'une autre technique d'imagerie fonctionnelle (IRMf par exemple), mais seulement en ajoutant à la technique de référence MNE une information à priori (mesure de corrélation) dans l'espace capteurs, affiner la localisation de l'activité cérébrale. Ces conclusions restent valables pour d'autres tâches comme l'imagination de mouvement de la jambe, impliquant d'autres zones corticales motrices.

**Revendications**

1. Méthode d'estimation de l'activité électrique au sein d'un tissu d'un sujet, ladite activité électrique étant associée à une tâche, effectuée, imaginée ou visualisée par le sujet lorsque celui-ci reçoit un stimulus, dans laquelle on réalise l'acquisition (110,210) d'une pluralité de signaux physiologiques grâce à une pluralité de capteurs disposés autour du tissu, ladite méthode étant **caractérisée en ce que** :

   - on calcule (120, 220) des coefficients de corrélation entre les différents signaux physiologiques et un signal représentatif dudit stimulus ;
   - on calcule (130, 230) la matrice de covariance des signaux physiologiques sur une fenêtre temporelle ;
   - on pondère (140,240) les coefficients de la matrice de covariance à l'aide des coefficients de corrélation, de sorte à pénaliser, en termes de rapport signal sur bruit, les signaux physiologiques qui sont faiblement corrélés avec le stimulus, la pénalisation diminuant les coefficients relatifs aux signaux physiologiques faiblement corrélés avec le stimulus, lorsque le stimulus est présent dans la fenêtre temporelle et/ou augmentant ces coefficients, lorsque celui-ci est absent de la fenêtre temporelle ;
   - on estime (150, 250) l'activité électrique, en au moins un point du tissu, à partir des signaux physiologiques et de la matrice de covariance ainsi pondérée.

2. Méthode d'estimation de l'activité électrique au sein d'un tissu selon la revendication 1, **caractérisée en ce que** l'estimation est basée sur un critère MNE.

3.  Méthode d'estimation de l'activité électrique au sein d'un tissu selon la revendication 2, **caractérisée en ce que** la matrice de covariance est une matrice de covariance de bruit calculée sur une fenêtre temporelle où le stimulus est absent et que l'activité électrique dans une pluralité de zones élémentaires du tissu est estimée au moyen de :

$$\hat{\mathbf{x}}(t) = \mathbf{A}^{T} \left( \mathbf{A}\mathbf{A}^{T} + p^{-1}\tilde{\mathbf{C}}(t) \right)^{-1} \mathbf{y}(t)$$

où $\hat{\mathbf{x}}(t)$ est un vecteur représentant l'activité électrique dans les différentes zones élémentaires, $\mathbf{y}(t)$ est un vecteur représentant les signaux physiologiques acquis par les capteurs, $\mathbf{A}$ est une matrice donnant la réponse des capteurs pour des sources de puissance unitaire situées dans les différentes zones élémentaires, $p$ est la puissance réelle de ces sources et C(t) est la matrice de covariance de bruit pondérée.

4.  Méthode d'estimation de l'activité électrique au sein d'un tissu selon la revendication 3, **caractérisée en ce que** les coefficients de matrice de covariance de bruit pondérée sont obtenus à partir de la matrice de covariance de bruit au moyen de la relation suivante :

$$\tilde{C}_{ij}(t) = C_{ij} \text{ pour } i = 1,...,N, j = 1,...,N, i \neq j$$

$$\tilde{C}_{ii}(t) = \frac{C_{ii}}{1 + \gamma^2 \chi_i^2(t)} \text{ pour } i = 1,...,N$$

où les coefficients $\tilde{C}_{ij}(t)$, $i = 1,..., N$, $j = 1,...,N$, sont les coefficients de la matrice de covariance de bruit pondérée, les coefficients $C_{ij}$, $i=1,...,N$, $j=1,...,N$ sont les coefficients de la matrice de covariance de bruit, $N$ est le nombre de capteurs, $\gamma$ est une constante réelle prédéterminé et $\chi_i(t)$, $i = 1,...,N$, sont les coefficients de corrélation des signaux physiologiques acquis par les différents capteurs avec le signal représentatif du stimulus.

5.  Méthode d'estimation de l'activité électrique au sein d'un tissu selon la revendication 4, **caractérisée en ce que** les coefficients de corrélation $\chi_i(t)$, $i = 1,...,N$ font l'objet d'une normalisation préalablement à la pondération des coefficients de la matrice de covariance de bruit.

6.  Méthode d'estimation de l'activité électrique au sein d'un tissu selon la revendication 1, **caractérisée en ce que** la méthode d'estimation utilise une formation de faisceau pour une pluralité de zones élémentaires du tissu et pour une pluralité de directions.

7.  Méthode d'estimation de l'activité électrique au sein d'un tissu selon la revendication 6, **caractérisée en ce que** la matrice de covariance est calculée sur une fenêtre temporelle où le stimulus est présent et que l'activité électrique dans une chaque zone élémentaire du tissu est estimée au moyen de :

$$\hat{x}_{m,k}(t) = \tilde{\mathbf{D}}(t)^{-1} \mathbf{L}_{m,k} \left( \mathbf{L}_{m,k} \tilde{\mathbf{D}}(t)^{-1} \mathbf{L}_{m,k} \right)^{-1} \mathbf{y}(t)$$

où $\hat{x}_{m,k}(t)$ représente l'activité électrique dans la zone élémentaire située en un point $\mathbf{r}_m$ et dans la direction $\mathbf{u}_k$, $\mathbf{y}(t)$ est un vecteur représentant les signaux physiologiques acquis par les capteurs, $\mathbf{L}_{m,k}$ est un vecteur de taille $N$ donnant la réponse des capteurs lorsqu'une source de puissance unitaire se trouve au point $\mathbf{r}_m$ et est orientée dans la direction $\mathbf{u}_k$, et où $\tilde{\mathbf{D}}(t)$ est la matrice de covariance pondérée.

8.  Méthode d'estimation de l'activité électrique au sein d'un tissu selon la revendication 7, **caractérisée en ce que** les coefficients de matrice de covariance pondérée sont obtenus à partir de la matrice de covariance au moyen de la relation suivante :

$$\tilde{D}_{ij}(t) = D_{ij} \left| \chi_i(t) \right| \left| \chi_j(t) \right| \quad i = 1, ..., N, \, j = 1, ..., N$$

où les coefficients $\tilde{D}_{ij}(t)$, $i=1,...,N$, $j=1,...,N$, sont les coefficients de la matrice de covariance pondérée, les coefficients $D_{ij}$, $i=1,...,N$, $j=1,...,N$ sont les coefficients de la matrice de covariance, $N$ est le nombre de capteurs et $\chi_i(t)$, $i=1,...,N$, sont les coefficients de corrélation des signaux physiologiques acquis par les différents capteurs avec le signal représentatif du stimulus.

9. Méthode d'estimation de l'activité électrique au sein d'un tissu selon l'une des revendications précédentes, **caractérisée en ce que** les coefficients de corrélation sont obtenus en effectuant (310) une transformée temps-fréquence ou temps-échelle de chaque signal physiologique pour obtenir une pluralité de composantes fréquentielles ($Y_f(t)$) de ce signal en fonction du temps, en calculant (320) les coefficients de Pearson ($R_f(t)$) entre lesdites composantes fréquentielles et le signal représentatif du stimulus, le coefficient de corrélation ($\chi(t)$) relatif à un signal physiologique étant déterminé à partir desdits coefficients de Pearson obtenus pour ce signal.

10. Méthode d'estimation de l'activité électrique au sein d'un tissu selon la revendication 9, **caractérisée en ce que** le coefficient de corrélation ($\chi(t)$) relatif à un signal physiologique est obtenu (330) comme la valeur extrémale des coefficients de Pearson pour les différentes composantes fréquentielles de ce signal.

acquisition des signaux physiologiques $y_i(t)$ du tissu par les capteurs $i = 1, ..., N$ — 110

calcul des coefficients de corrélation $\chi_n(t)$ des signaux physiologiques avec le signal de stimulus $\eta(t)$ — 120

calcul de la matrice $\mathbf{C}$ de covariance de bruit — 130

calcul de la matrice $\tilde{\mathbf{C}}(t)$ de covariance de bruit pondérée à partir de la matrice $\mathbf{C}$ et des coefficients de corrélation $\chi_n(t)$ — 140

estimation de l'activité électrique dans le tissu
$$\hat{\mathbf{x}}(t) = \mathbf{A}^T \left( \mathbf{A}\mathbf{A}^T + p^{-1}\tilde{\mathbf{C}}(t) \right)^{-1} \mathbf{y}(t)$$
— 150

# FIG. 1

acquisition des signaux physiologiques $y_i(t)$
du tissu par les capteurs $i = 1, ..., N$

210

calcul des coefficients de corrélation $\chi_n(t)$ des signaux
physiologiques avec le signal de stimulus $\eta(t)$

220

calcul de la matrice $\mathbf{D}$ de covariance des
signaux physiologiques

230

calcul de la matrice $\tilde{\mathbf{D}}(t)$ de covariance pondérée
à partir de la matrice $\mathbf{D}$ et des coefficients de corrélation $\chi_n(t)$

240

estimation de l'activité électrique dans le tissu
$\hat{x}_{m,k}(t) = \tilde{\mathbf{D}}(t)^{-1} \mathbf{L}_{m,k} \left( \mathbf{L}_{m,k} \tilde{\mathbf{D}}(t)^{-1} \mathbf{L}_{m,k} \right)^{-1} \mathbf{y}(t)$

250

# FIG. 2

transformée temps-fréquence ou temps-échelle
du signal physiologique $y(t)$ : obtention de $Y_f(t)$

310

calcul des coefficients de Pearson des composantes
fréquentielles $Y_f(t)$ avec le signal de stimulus

$$R_f(t) = \frac{1}{\sigma_\eta \cdot \sigma_{Y_f}} \int\limits_{[t,t+T]} Y_f(u)\big(\eta(u) - \bar{\eta}\big)\,du$$

320

calcul du coefficient de corrélation $\chi(t)$

$$\chi(t) = \underset{f}{extrem}\big[R_f(t)\big]$$

330

# FIG. 3

FIG. 4A

FIG. 4B

**Europäisches Patentamt**
**European Patent Office**
**Office européen des brevets**

## RAPPORT DE RECHERCHE EUROPEENNE

Numéro de la demande

EP 15 17 8478

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | ARTHUR K LIU ET AL: "Spatiotemporal imaging of human brain activity using functional MRI constrained magnetoencephalography data: Monte Carlo simulations", PROCEEDINGS OF THE NATIONAL ACADEMY OF SCIENCES OF THE UNITED STATES OF AMERICA NEUROBIOLOGY, vol. 95, no. 6, juillet 1998 (1998-07), pages 8945-8950, XP055179344, * page 8946 - page 8949 * ----- | 1-10 | INV. G06F17/18 G06F3/01 A61B5/04 |
| Y | EP 2 679 151 A1 (COMMISSARIAT ENERGIE ATOMIQUE [FR]) 1 janvier 2014 (2014-01-01) * page 4 - page 6 * ----- | 1-10 | |
| A | G VAN HOEY ET AL: "Beamforming Techniques Applied in EEG Source Analysis", PROCEEDINGS OF THE PRORISC CIRCUITS, SYSTEMS AND SIGNAL PROCESSING, 1999, pages 545-549, XP055179214, * le document en entier * ----- | 1-10 | |
| A | CN 103 699 226 A (UNIV TIANJIN) 2 avril 2014 (2014-04-02) * le document en entier * ----- | 1-10 | DOMAINES TECHNIQUES RECHERCHES (IPC) A61B G06F |
| A | MOLINS A ET AL: "Quantification of the benefit from integrating MEG and EEG data in minimum @?2-norm estimation", NEUROIMAGE, ACADEMIC PRESS, ORLANDO, FL, US, vol. 42, no. 3, septembre 2008 (2008-09), pages 1069-1077, XP026446469, ISSN: 1053-8119 [extrait le 2008-06-14] * le document en entier * ----- | 1-10 | |

Le présent rapport a été établi pour toutes les revendications

| Lieu de la recherche | Date d'achèvement de la recherche | Examinateur |
|---|---|---|
| Berlin | 18 novembre 2015 | Huguet Serra, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 15 17 8478

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-11-2015

| Document brevet cité au rapport de recherche | Date de publication | Membre(s) de la famille de brevet(s) | Date de publication |
|---|---|---|---|
| EP 2679151 A1 | 01-01-2014 | EP 2679151 A1<br>FR 2992543 A1<br>US 2014107464 A1 | 01-01-2014<br>03-01-2014<br>17-04-2014 |
| CN 103699226 A | 02-04-2014 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Beamforming and its application to brain connectivity. **A. FUCHS.** Handbook of Brain Connectivity. Springer Verlag, 2007, 357-378
- **O. HAUK et al.** Comparison of noise-normalized minimum norm estimates of MEG analysis using multiple resolution metrics. *Neuroimage,* 2011, vol. 54, 1966-1974
- **MONTE CARLO.** *Proceedings of the National Academy of Sciences of the United States of America,* Juillet 1998, vol. 95 (15), 8945-8950
- **J. VRBA et al.** Signal processing in magnetoencephalography. *Methods,* 2001, vol. 25, 249-271